# EUROPÄISCHE PATENTSCHRIFT

(11) **EP 2 593 381 B1**
(45) Veröffentlichungstag und Bekanntmachung des Hinweises auf die Patenterteilung: **07.09.2016**
(21) Anmeldenummer: 11733677.6
(22) Anmeldetag: 14.07.2011
(51) Int. Cl.: B65D 77/00

(54) **MEDIZINISCHE VERBRAUCHSGÜTERVERPACKUNG**
PACKAGE FOR MEDICAL CONSUMABLE PRODUCTS
EMBALLAGE POUR PRODUITS CONSOMMABLES MÉDICAUX

(30) Priorität: 16.07.2010 DE 202010010350 U
(43) Veröffentlichungstag der Anmeldung: 22.05.2013
(73) Patentinhaber: Sarstedt AG & Co. KG, 51588 Nürnbrecht (DE)
(72) Erfinder: RIGLING, Joachim, 75382 Althengstett (DE); RIGLING, Tobias, 75382 Althengstett (DE)
(74) Vertreter: Wagner, Kilian
(86) Internationale Anmeldenummer: PCT/EP2011/062079
(87) Internationale Veröffentlichungsnummer: WO 2012/007554

(56) Entgegenhaltungen:
- EP-A1- 1 149 601
- DE-A1-102005 036 329
- DE-A1-102006 022 018
- GB-A- 2 194 514
- US-A- 5 152 394
- "NEW PACKAGING DESIGN FOR MOBILE PRODUCTS", IBM TECHNICAL DISCLOSURE BULLETIN, INTERNATIONAL BUSINESS MACHINES CORP. (THORNWOOD), US, Bd. 40, Nr. 10, 1. Oktober 1997 (1997-10-01), Seite 47/48, XP000739417, ISSN: 0018-8689

## Beschreibung

Die Erfindung betrifft eine medizinische Verbrauchsgüterverpackung, umfassend einen, insbesondere an einer Oberseite, öffenbaren Umkarton, in dem eine Anzahl von medizinischen Verbrauchsgütern, wie beispielsweise Einwegkanülen, aufgenommen sind.

Es ist bekannt medizinische Verbrauchsgüter wie Einwegkanülen in an einer Oberseite öffenbaren Umkartons zu verpacken. Nach dem bestimmungsgemäßen Gebrauch der Verbrauchsgüter werden diese nicht unmittelbar dem normalen Restmüll zugeführt sondern stattdessen in spezielle Kunststoff-Abfallbehälter eingesteckt, die so beschaffen sind, dass die häufig spitzen- und/oder scharfkantigen medizinischen Verbrauchsgüter beim bestimmungsgemäßen Gebrauch des Kunststoff-Abfallbehälters diesen nicht beschädigen bzw. durchstoßen, wodurch ein Gefährdung von medizinischem Personal hinsichtlich Infektionen, Verletzungen etc. vermieden wird. Die vorgenannten Kunststoff-Abfallbehälter werden separat zu den die medizinischen Verbrauchsgüter beinhaltenden Umkartons geliefert, wozu es einer separaten Logistik sowie zusätzlicher Umverpackungen bedarf, was zu vergleichsweise hohen Lager- und Transportkosten führt.

Aus der US 5 152 394 A ist ein Abfallbehälter für medizinische Verbrauchsgüter (Spritzen) bekannt, welcher ein wannenförmiges Unterteil sowie einen Deckel aufweist. Bei Anlieferung, d.h. vor Ingebrauchnahme enthält das Abfallbehältnis unverbrauchte, d.h. neue Verbrauchsgüter.

Zum weiteren Stand der Technik werden die DE 10 2005 036 329 A1, die EP 1 149 601 A1, die DE 10 2006 022 018 A1 sowie die GB 2 194 514 A genannt.

Ausgehend von dem vorgenannten Stand der Technik liegt der Erfindung die Aufgabe zugrunde, Lager- und Transportkosten für medizinische Verbrauchsgüter sowie für Kunststoff-Abfallbehälter für diese Verbrauchsgüter zu reduzieren. Bevorzugt soll eine entsprechend optimierte medizinische Verbrauchsgüterverpackung komfortabel handhabbar sein.

Diese Aufgabe wird bei einer gattungsgemäßen medizinischen Verbrauchsgüterverpackung dadurch gelöst, dass in dem Verbrauchsgüter-Umkarton ein die medizinischen Verbrauchsgüter aufnehmender wannenartigen Kunststoff-Abfallbehälter zur späteren Aufnahme der Verbrauchsgüter nach deren Benutzung angeordnet ist, der mit einer mittels eines zwischen einer geschlossenen Bodenseite des Kunststoff-Abfallbehälters und der Oberseite des Umkartons angeordneten, eine Einstecköffnung für benutzte medizinische Verbrauchsgüter aufweisenden Deckels verschließbaren Öffnungsseite einer Unterseite des Umkartons und mit der geschlossenen Bodenseite der Oberseite des Umkartons zugewandet ist und/oder, dadurch dass in dem Umkarton ein die medizinischen Verbrauchsgüter aufnehmender wannenartigen Kunststoff-Abfallbehälter zur späteren Aufnahme der Verbrauchsgüter nach deren Benutzung angeordnet ist, der eine Öffnungsseite aufweist, die mittels eines im Umkarton aufgenommenen, eine Einstecköffnung für benutzte medizinische Verbrauchsgüter aufweisenden Deckels verschließbar ist.

Unter dem Merkmal "wannenartig" wird dabei eine Behälterform verstanden, die neben einer geschlossenen Bodenfläche eine umfangsgeschlossene Umfangswand aufweist, die die medizinischen Verbrauchsgüter umschließt und auf die, vorzugsweise stirnseitig der ebenfalls in der Karton-Umverpackung enthaltene Deckel aufsetzbar ist.

Vorteilhafte Weiterbildungen der Erfindung sind in den Unteransprüchen angegeben. In den Rahmen der Erfindung fallen sämtliche Kombinationen von zumindest zwei in der Beschreibung, den Ansprüchen und/oder in den Figuren offenbarten Merkmalen.

Der Erfindung liegt der Gedanke zugrunde, den wannenartigen Kunststoff-Abfallbehälter, der geeignet und bestimmt ist die medizinischen Verbrauchsgüter nach deren Benutzung sicher aufzunehmen, zusätzlich zu den (unbenutzten) medizinischen Verbrauchsgütern in dem Umkarton vorzusehen, und zwar derart, dass die medizinischen Verbrauchsgüter in dem wannenartigen Kunststoff-Abfallbehälter aufgenommen sind. Bevorzugt befindet sich somit der Kunststoff-Abfallbehälter bzw. dessen umfangsgeschlossene Umfangswand zwischen dem Umkarton und den medizinischen Verbrauchsgütern. Der Deckel, der unlösbar mit dem Kunststoff-Abfallbehälter zum Verschließen der Öffnungsseite desselben verbindbar ist, ist ebenfalls in dem Umkarton aufgenommen.

Mit der Erfindung werden gleich mehrere Vorteile auf einmal realisiert. Ein Hauptvorteil besteht darin, dass das Transport- und Lagervolumen für Kunststoff-Abfallbehälter und medizinische Verbrauchsgüter bei einer nach dem Konzept der Erfindung ausgebildeten Verbrauchsgüterverpackung wesentlich reduziert ist. Darüber hinaus wird die Versand- und Lagerlogistik vereinfacht. Zudem wird aufgrund der erfinderischen Maßnahme bzw. Anordnung ein höherer Produktschutz, d.h. ein erhöhter Schutz der medizinischen Verbrauchsgüter vor Beschädigung durch Stoßeinwirkung beim Transport reduziert, da die Umfangswand sowie der Boden des wannenförmigen Verpackungsbehälters schützt. Ferner stabilisiert der Kunststoff-Abfallbehälter die Umverpackung, wodurch bei Bedarf auf eine dünnere Kartonage zur Ausbildung des Umkartons zurückgegriffen werden kann. Zudem kann die Kartonumverpackung für die medizinischen Verbrauchsgüter, die gleichzeitig eine Umverpackung für den Abfallbehälter darstellt, nach Entnahme des Abfallbehälters zur Aufbewahrung der medizinischen Verbrauchsgüter dienen, welche nach ihrem Gebrauch durch die Einstecköffnung im am Kunststoff-Abfallbehälter festgelegten Deckel in diesen überführt werden können. Durch die Erfindung wird also ein neuartiges System bzw. eine neuartige Verpackung bereitgestellt, die gleichzeitig die medizinische Verbrauchsgüter als auch den Kunststoff-Abfallbehälter samt Deckel umfasst, wobei der Kunststoff-Abfallbehälter selbst einen Bestandteil der Verpackung bildet und der zudem die Karton-Umverpackung, die nach Entnahme des Abfallbehälters zur Aufbewahrung der unbenutzten medizinischen Verbrauchsgüter dienen kann stabilisiert.

Ganz besonders bevorzugt ist eine Ausführungsvariante der medizinischen Verbrauchsgüterverpackung, bei der der wannenartige Kunststoff-Abfallbehälter mit seiner Bodenseite in Richtung einer öffenbaren, insbesondere aufklappbaren, Umkarton-Oberseite orientiert ist, also folglich mit seiner mittels des Deckels verschließbaren Öffnungsseite nach unten in Richtung unterer Seite des Umkartons gerichtet ist, wobei der Deckel des Kunststoff-Abfallbehälters zwischen Bodenseite des Kunststoff-Abfallbehälters und öffenbarer Umkarton-Oberseite angeordnet ist, vorzugsweise unmittelbar auf der Außenseite des Bodens des Kunststoff-Abfallbehälters aufliegt. Eine derartige Ausführungsvariante hat den Vorteil, dass der Deckel und der Kunststoff-Abfallbehälter nach Öffnen des Umkartons nach oben aus letzterem herausgezogen werden können ohne dabei die unbenutzten medizinischen Verbrauchsgüter aus dem Umkarton entfernen zu müssen. Diese verbleiben automatisch im geöffneten und insbesondere wiederverschließbaren Umkarton.

Hinsichtlich der Realisierung der Öffenbarkeit des Umkartons gibt es eine Vielzahl von Möglichkeiten. Bevorzugt ist der Umkarton wieder verschließbar. Ganz besonders bevorzugt ist zum Öffnen des Umkartons, insbesondere an dessen Oberseite, ein aufklappbarer, insbesondere mit einer nach innen einsteckbaren Lasche versehener Umkarton-Deckel vorgesehen, der noch weiter bevorzugt Bestandteil eines einstückigen Kartonzuschnittes ist. Alternativ sind jedoch auch andere an sich bekannte Öffnungsmechanismen, wie das Vorsehen mindestens einer Schwächungslinie, insbesondere einer perforierten Linie zum Ablösen eines Umkartonabschnittes od. dgl. Mechanismus realisierbar.

Grundsätzlich sind jedoch auch im Vergleich zu dem zuvor vorbeschriebenen bevorzugten Ausführungsbeispiel alternative Anordnungen des Kunststoff-Abfallbehälters in der Verbrauchsgüterverpackung realisierbar. So ist es beispielsweise möglich, den Kunststoff-Abfallbehälter mit seiner Bodenseite auf die Unterseite (Bodenseite) des Unkartons aufzustellen, so dass die Öffnungsseite des Kunststoff-Behälters mit den darin befindlichen medizinischen Verbrauchsgütern nach oben weist. Bei einer derartigen Anordnung kann der Kunststoff-Abfallbehälter lediglich gemeinsam mit den medizinischen Verbrauchsgütern aus dem Unkarton entnommen werden, die dann wiederum bevorzugt in den Unkarton zurückentleert werden. Anstelle des Aufstellens des Kunststoff-Abfallbehälters unmittelbar auf einer Unterseite (Bodenseite bzw. Boden) des Unkartons kann zwischen Unkartonboden und Kunststoff-Abfallbehälter der Deckel zum Verschließen desselben vorgesehen werden. Auch ist es möglich den Deckel zwischen Öffnungsseite des Kunststoff-Abfallbehälters und Unkarton, insbesondere zwischen Öffnungsseite des Kunststoff-Abfallbehälters und Oberseite anzuordnen, wobei bevorzugt darauf zu achten ist, dass ein unbeabsichtigtes Verrasten des Deckels am Kunststoff-Abfallbehälter vermieden wird. Hierzu ist es beispielsweise denkbar den Deckel um 180° verdreht im Vergleich zur bestimmungsgemäßen Orientierung auf die Öffnungsseite aufzulegen. Ebenso ist es möglich den Deckel zwischen Umfangswand des Unkartons und Umfangswand des Kunststoff-Abfallbehälters anzuordnen.

Besonders bevorzugt ist der Deckel unlösbar mit dem Abfallbehälter verbindbar. Unter dem Merkmal "unlösbar verbindbar" wird verstanden, dass der Deckel derart an dem Kunststoff-Abfallbehälter, festlegbar ist, dass ein Ablösen nur durch erhebliche Kraftaufwendung, insbesondere unter Zerstörung mindestens eines Deckel- und/oder Abfallbehälterbereiches, insbesondere der Befestigungsmittel (z.B. Verrastung) möglich ist. Hierdurch wird die Sicherheit vor Verletzungen erhöht und ein unbeabsichtigtes Öffnen sicher vermieden.

Ganz besonders bevorzugt ist eine Ausführungsvariante, bei der der Umfangskarton, zumindest näherungsweise, quaderförmig konturiert ist, um somit das Verpackungsvolumen und Lagervolumen eines mehrere Verbrauchsgüterverpackungen umfassenden Verpackungs- bzw. Lagerloses weiter zu reduzieren. Ganz besonders zweckmäßig ist es, wenn die Umfangskontur bzw. Hüllkontur des Abfallbehälters, zumindest näherungsweise an die Umfangskontur des Unkartons angepasst ist, um zum einen das Unkartonvolumen möglichst vollständig auszunutzen und um somit keinen Raum zu verschenken und um eine optimale Stabilisierung des Unkartons zu gewährleisten, Besonders zweckmäßig ist also der Kunststoff-Abfallbehälter bzw. dessen Hüllkontur ebenfalls quaderförmig konturiert, wobei es denkbar ist, dass keine scharfkantigen, sondern gerundete Eckkonturen realisiert sind.

Wie eingangs angedeutet ist es bevorzugt, wenn die innerhalb des im Unkarton angeordneten wannenartigen Kunststoff-Abfallbehälter aufgenommenen medizinischen Verbrauchsgüter Kanülen und/oder Nadeln und/oder Schneiden umfassen oder als solche ausgebildet sind. Ganz besonders zweckmäßig sind die medizinischen Verbrauchsgüter scharfkantig und/oder spitz, wobei gegebenenfalls entsprechende Schutzkappen am jeweiligen Verbrauchsgut vorgesehen sein können.

Ganz besonders zweckmäßig ist es, wenn der Durchmesser und/oder die (Umfangs-) Kontur der Einstecköffnung an die Abmessungen der in der Verbrauchsgüterverpackung aufgenommenen Verbrauchsgüter angepasst ist, dass diese einzeln nach ihrem bestimmungsgemäßen Verbrauch durch die Einstecköffnung hindurchsteckbar sind. Gemeint ist aber der Durchmesser und/oder die Kontur der wirksamen Einstecköffnung, d.h. der maximalen Einstecköffnung im Falle des Vorsehens einer Einstecköffnung variabler und/oder sich beim Einstecken von Verbrauchsgütern ändernder Einstecköffriungsgeometrie.

In Abhängigkeit der Beschaffenheit der in der Verbrauchsgüterverpackung aufgenommenen medizinischen Verbrauchsgüter kann die Einstecköffnungskontur beispielsweise zum Abstreifen der Verbrauchsgüter ausgebildet sein. Dies ist insbesondere im Falle der Ausbildung der Verbrauchsgüter als Kanülen zweckmäßig.

Gegebenenfalls können am Deckel Mittel zum Öffnen und/oder Verschließen der Einstecköffnung vorgesehen sein. Beispielsweise kann hierzu ein Klapp- oder Schiebemechanismus realisiert sein, mittels dem die Einstecköffnung öffen- und/oder verschließbar, gegebenenfalls nach Befüllen des Kunststoff-Abfallbehälters dauerhaft verschließbar ist.

Wie bereits angedeutet kann die Einstecköffnung gemäß einer ersten Alternative eine unveränderliche Querschnittsöffnung aufweisen oder alternativ gemäß einer zweiten Alternative eine veränderliche Querschnitts- bzw. Umfangskontur. Im letztgenannten Fall wird dadurch bevorzugt bezweckt bzw. erreicht, dass die Querschnittskontur beim Einstecken eines Verbrauchsgutes elastisch vergrößert wird, woraufhin, zumindest näherungsweise, die ursprüngliche Querschnittskontur sich wieder einstellt, die so bemessen ist, dass ein Herausfallen der eingesteckten Verbrauchsgüter auch beim Umdrehen der Verbrauchsgüterverpackung sicher vermieden wird.

Um zu gewährleisten, dass mit den benutzten medizinischen Verbrauchsgütern eine Beschädigung bzw. ein Durchstechen der Umfangswand und/oder des Bodens des Kunststoff-Abfallbehälters möglich ist, ist in Weiterbildung der Erfindung mit Vorteil vorgesehen, dass die minimale Wandstärke, d.h. die Wandstärke an der dünnsten Stelle mindestens 1,25mm beträgt. Bevorzugt ist die Wandstärke aus einem Wertebereich zwischen 1,3mm und 2,0mm gewählt.

Weitere Vorteile, Merkmale und Einzelheiten der Erfindung ergeben sich aus der nachfolgenden Beschreibung bevorzugter Ausführungsbeispiele sowie anhand der Zeichnungen.

Diese zeigen in:
- Fig. 1:: eine Ansicht von schräg oben auf eine medizinische Verbrauchsgüterverpackung, umfassend einen oben geöffnet dargestellten Umkarton mit innenliegendem Deckel sowie einem darunter angeordneten nicht sichtbaren Kunststoff-Abfallbehälter, in welchem die ebenfalls nicht dargestellten medizinischen Verbrauchsgüter aufgenommen sind,
- Fig. 2:: die Darstellung der Verbrauchsgüterverpackung gemäß Fig. 1 mit teilweise nicht dargestellter Umkarton-Umfangswand zur Sichtbarmachung des Kunststoff-Abfallbehälters,
- Fig. 3:: eine aufgebrochene Darstellung der Verbrauchsgüterverpackung zur Darstellung des Innenlebens,
- Fig. 4:: eine hälftig aufgeschnittene Darstellung der Verbrauchsgüterverpackung ohne Deckelverschlussmechanismus und

- Fig. 5:: eine weitere alternative Darstellung der geschnittenen Verbrauchsgüterverpackung.

In den Figuren sind gleiche Elemente und Elemente mit der gleichen Funktion mit den gleichen Bezugszeichen gekennzeichnet.

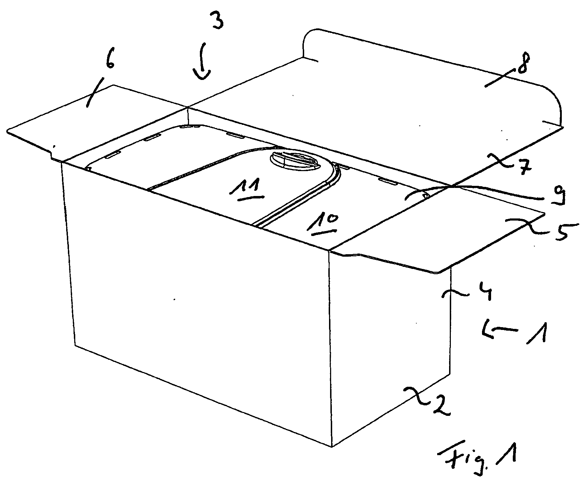

In Fig. 1 ist ein bevorzugtes Ausführungsbeispiel einer nach dem Konzept der Erfindung ausgebildeten medizinischen Verbrauchsgüterverpackung 1 gezeigt, Diese umfasst einen quaderförmig konturierten Umkarton 2 aus einem Kartonmaterial. Der Umkarton 2 weißt eine öffen- und bei dem gezeigten Ausführungsbeispiel wiederverschließbare Oberseite 3 auf. Hierzu umfasst der einteilige Kartonzuschnitt 4 zwei obere seitliche, klappbare Deckelabschnitte 5, 6 die mit einem rechtwinklig dazu verlaufenden, ebenfalls klappbaren Verschlussdeckel 7 im geschlossenen Zustand überdeckbar sind. Der Verschlussdeckel 7 umfasst eine randseitige äußere Lasche 8, die zum Einstecken und in das Innere des Umkartons und damit Verschließen bzw. Fixieren des Verschlussdeckels 7 im geschlossenen Zustand bestimmt ist.

Innerhalb des Umkartons 2 befindet sich ein aus Kunststoff ausgebildeter Deckel 9 für einen ebenfalls im Umkarton befindlichen wannenartigen Kunststoff-Abfallbehälter, in welchem wiederum die medizinischen Verbrauchsgüter aufgenommen sind. Zu erkennen ist die bei bestimmungsgemäßer Verwendung nach außen gerichtete Außenseite 10 des Deckels 9 mit einer verschwenkbaren Verschlussklappe 11 zum Verschließen einer darunter befindlichen Einstecköffnung.

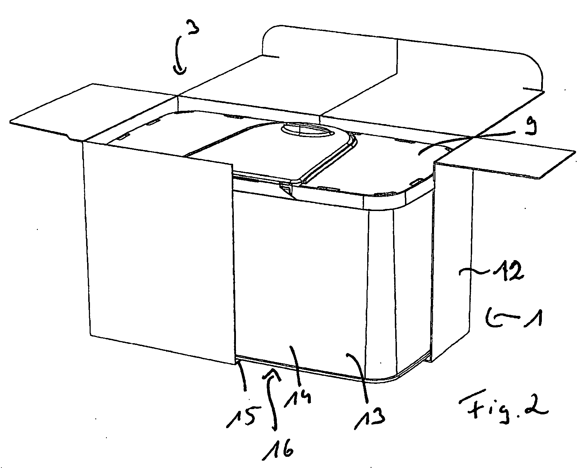

In Fig. 2 ist zur Darstellung des "Innenlebens" der Verbrauchsgüterverpackung 1 ein Teil der umfangsgeschlossenen Umfangswand 12 des Umkartons 2 nicht gezeigt. Zur erkennen ist der unterhalb des aus Kunststoff ausgebildeten Deckels 9 befindliche Kunststoff-Abfallbehälter 13, der in dem gezeigten Ausführungsbeispiel aus 1,4mm ausgebildet ist. Bei dem Kunststoff-Abfallbehälter handelt es sich um ein Kunststoffspritzgussteil. Der Kunststoff-Abfallbehälter 13 weist neben einer umfangsgeschlossenen Umfangswand 14 eine der Oberseite 3 des Umkartons sowie dem Deckel 9 zugewande Bodenseite (Bodenabschnitt) auf, die in der Darstellung gemäss Fig. 2 vom Deckel verdeckt ist. Mit der der Bodenseite gegenüberliegenden, nach unten gerichteten Öffnungsseite 16 liegt der Abfallbehälter 13 auf einer Unterseite 15 (Bodenseite bzw. Boden) des Umkartons 2 auf. Diese ist mittels des Deckels 9 verschließbar.

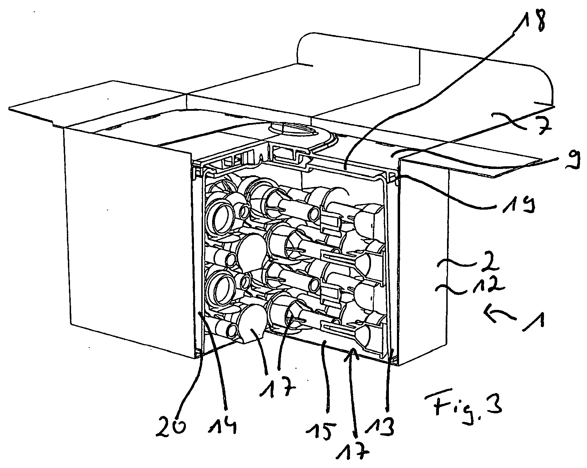

In Fig. 3 ist die Verbrauchsgüterverpackung 1 in einem aufgebrochenen Zustand gezeigt. Zu erkennen sind eine Vielzahl von in dem Kunststoff-Abfallbehälter 13 aufgenommen medizinischen Verbrauchsgüter 17, bei denen es sich in dem gezeigten Ausführungsbeispiel um Einwegkanülen, bzw. für Diabeteskranke handelt, die mit einer Schutzkappe geschützt sind. Die Verbrauchsgüter 17 sind umgeben von der Umfangswand 14 des Kunststoff-Abfallbehälters 13 und liegen auf der Innenseite der Unterseite 15 (Boden) des Unkartons 2 auf. Die Umfangswand 14 hat somit eine zusätzliche Schutzfunktion für die medizinischen Verbrauchsgüter 17 und stabilisiert zudem die lediglich wenig beabstandete Umfangswand 12 des Umkartons 2.

In Fig. 3 ist gut die nach unten gerichtete Öffnungsseite 16 des Kunststoff-Abfallbehälters 13 zu erkennen sowie die nach oben gerichtete Bodenseite 18 des Kunststoff-Abfallbehälters zwischen der und dem Verschlussdeckel 7 sich im geschlossenen Zustand der medizinischen Verbrauchsgüterverpackung 1 der Deckel 9 befindet, welcher lose auf dem Kunststoff-Abfallbehälter 13 aufliegt.

Zu erkennen ist eine umlaufende, untere Fixiernut 19 am Deckel 9, die axial in einem Bereich radial zwischen der Umfangswand 14 des Kunststoff-Abfallbehälters 13 und der Umfangswand 12 des Umkartons 2 eingeschoben ist. Die Fixiernut 19 dient zum Fixieren bzw. Aufsetzen auf die die Öffnungsseite 16 bzw. die Öffnung des Kunststoff-Abfallbehälters 13 begrenzende nach unten gerichtete Stirnkante des Kunststoff-Abfallbehälters 13. Die Fixiernut 19 wirkt derart mit der Stirnkante 20 zusammen, dass der Deckel 9 nicht zerstörungsfrei vom Kunststoff-Abfallbehälter 13 abgenommen werden kann, wenn der Deckel 9 einmal korrekt fixiert wurde.

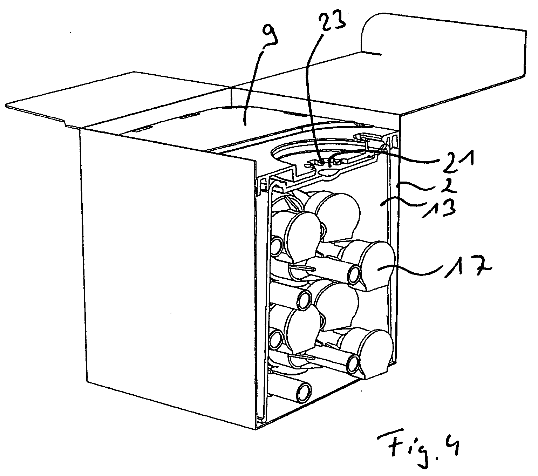

In Fig. 4 ist die Verschlussklappe zum Verschließen einer Einstecköffnung 21 im Deckel 9 nicht dargestellt. Die Geometrie der Einstecköffnung 21 ist derart auf die medizinischen Verbrauchsgüter 17 abgestimmt, dass diese, ggf. nach Entfernen einer Schutzkappe, nach der bestimmungsgemäßen Verwendung von außen durch die Einstecköffnung 21 in den Kunststoff-Abfallbehälter 13 überführbar sind. In dem gezeigten Ausführungsbeispiel ist die Einstecköffnung 21 begrenzt von nach radial innen weisenden, in axialer Richtung nach unten elastisch verformbaren angespritzten Fortsätzen 23, welche sich beim Einstecken der gebrauchten Verbrauchsgüter 17 elastisch verformen und danach wieder in die dargestellten Lage zurückverformen, um somit ein Herausfallen von einmal eingesteckten Verbrauchsgütern sicher zu vermeiden.

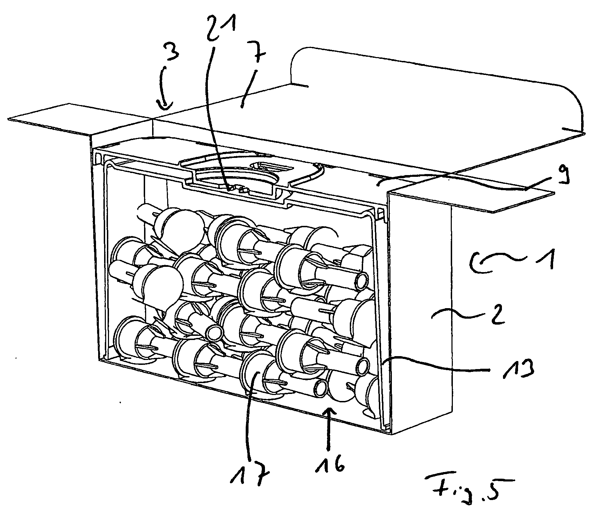

Fig. 5 zeigt eine Längsschnittansicht durch die Verbrauchsgüterverpackung 1. Zum Entnehmen des Kunststoff-Abfallbehälters 13 samt Deckel 9 wird zunächst die Oberseite 3 durch Aufklappen der Deckelabschnitte 5, 6 sowie des Verschlussdeckels 7 geöffnet. Daraufhin wird der Deckel 9 entnommen, ggf. unmittelbar zusammen mit dem Kunststoff-Abfallbehälter 13. Alternativ kann zunächst der Deckel 9 und danach der Kunststoff-Abfallbehälter 13 nach oben herausgezogen werden. Da der Kunststoff Abfallbehälter13 mit seiner Öffnungsseite 16 nach unten weist verbleiben die medizinischen Verbrauchsgüter 17 in dem als Vorratsbehältnis dienenden Umkarton 2. Daraufhin kann der Deckel 9, vorzugsweise unlösbar die Öffnungsseite 16 verschließend am Kunststoff-Abfallbehälter 13 fixiert werden. Nachdem der Kunststoff-Abfallbehälter 13 vollständig mit gebrauchten Verbrauchsgütern 17 gefüllt ist, kann dieser seiner Entsorgung zugeführt werden.

### Bezugszeichen

- 1: med. Verbrauchsgüterverpackung
- 2: Umkarton
- 3: Oberseite
- 4: Kartonzuschnitt
- 5: Deckelabschnitt
- 6: Deckelabschnitt
- 7: Verschlussdeckel
- 8: Lasche
- 9: Deckel
- 10: Außenseite
- 11: Verschlusskappe
- 12: Umfangswand des Umkartons
- 13: Kunststoff-Abfallbehälter
- 14: Umfangswand des Kunststoff-Abfallbehälters
- 15: Unterseite des Umkartons
- 16: Öffnungsseite des Kunststoff-Abfallbehälters
- 17: med. Verbrauchsgüter
- 18: Bodenseite des Kunststoff-Abfallbehälters
- 19: Fixiernut
- 20: Stirnkante
- 21: Einstecköffnung
- 22: Fortsätze
- 23: Fortsätze

## Patentansprüche

1. Medizinische Verbrauchsgüterverpackung (1), umfassend einen an einer Oberseite (3) öffenbaren Umkarton (2), in dem eine Anzahl von medizinischen Verbrauchsgütern (17), insbesondere Einwegkanülen, aufgenommen sind, wobei in dem Umkarton (2) ein die medizinischen Verbrauchsgüter (17) aufnehmender wannenartigen Kunststoff-Abfallbehälter (13) zur späteren Aufnahme der Verbrauchsgüter (17) nach deren Benutzung angeordnet ist, der mit einer mittels eines zwischen einer geschlossenen Bodenseite (18) des Kunststoff-Abfallbehälters (13) und der Oberseite (3) des Umkartons (2) angeordneten, eine Einstecköffnung (21) für benutzte medizinische Verbrauchsgüter (17) aufweisenden Deckels (9) verschließbaren Öffnungsseite (16) einer Unterseite (15) des Umkartons (2) und mit der geschlossenen Bodenseite (18) der Oberseite (3) des Umkartons (2) zugewandet ist.

2. Verbrauchsgüterverpackung nach Anspruch 1,
**dadurch gekennzeichnet,**
**dass** an dem Deckel (9) und/oder dem Kunststoff-Abfallbehälter (13), vorzugsweise als Rastverbindung ausgebildete, Mittel zum unlösbaren Festlegen des Deckels (9) am Kunststoff-Abfallbehälter (13) und dadurch Verschießen der Öffnungsseite (16) des Kunststoff-Abfallbehälters (13) vorgesehen sind.

3. Verbrauchsgüterverpackung nach einem der vorhergehenden Ansprüche,
**dadurch gekennzeichnet,**
**dass** der Umkarton (2) quaderförmig konturiert ist und/oder dass der Kunststoff-Abfallbehälter (13) quaderförmig konturiert ist, ggf. mit gerundeten Ecken.

4. Verbrauchsgüterverpackung nach einem der vorhergehenden Ansprüche,
**dadurch gekennzeichnet,**
**dass** die medizinischen Verbrauchsgüter (17) Kanülen und/oder Nadeln und/oder Schneiden umfassen und/oder scharfkantig und/oder spitz sind.

5. Verbrauchsgüterverpackung nach einem der vorhergehenden Ansprüche,
**dadurch gekennzeichnet,**
**dass** der Durchmesser und/oder die Kontur der wirksamen Einstecköffnung (21) an die Abmessungen der Verbrauchsgüter (17), insbesondere nach Abziehen einer Schutzkappe, derart angepasst ist, dass diese einzeln nach deren Benutzung durch die Einstecköffnung (21) hindurchsteckbar sind.

6. Verbrauchsgüterverpackung nach einem der vorhergehenden Ansprüche,
**dadurch gekennzeichnet,**
**dass** die Einstecköffnungskontur zum Abstreifen der Verbrauchsgüter (17) ausgebildet ist.

7. Verbrauchsgüterverpackung nach einem der vorhergehenden Ansprüche,
**dadurch gekennzeichnet,**
**dass** Mittel zum Öffnen und/oder Verschließen der Einstecköffnung (21) am Deckel (9) vorgesehen sind.

8. Verbrauchsgüterverpackung nach Anspruch 7,
**dadurch gekennzeichnet,**
**dass** die Einstecköffnung (21) von einer durch Einstecken der Verbrauchsgüter (17) elastisch verformbaren Querschnittskontur umgeben ist, die ein Herausfallen der eingesteckten Verbrauchsgüter (17) bei nach unten gerichteter Einstecköffnung (21) vermeidet.

9. Verbrauchsgüterverpackung nach einem der vorhergehenden Ansprüche,
**dadurch gekennzeichnet,**
**dass** die minimale Wandstärke der Umfangswand (14) und/oder des Bodens des Kunststoff-Abfallbehälters (13) mindestens 1,25mm beträgt und/oder aus einem Wertebereich zwischen 1,3mm und 2,0mm gewählt ist.

## Claims

1. A medical supplies packaging (1), comprising an outer box (2) openable on an upper side (3), in which a number of medical supplies (17), in particular disposable cannulas, are accommodated,
wherein a tub-like plastic waste container (13) accommodating the medical supplies (17) is arranged in the outer box (2) for later accommodating the supplies (17) after use thereof, wherein an opening side (16) of said container, which can be closed by means of a lid (9) that is arranged between a closed bottom face (18) of the plastic waste container (13) and the upper face (3) of the outer box (2) and comprises an insertion opening (21) for used medical supplies (17), faces a lower face (15) of the outer box (2) and the closed bottom face (18) of said container faces the upper face (3) of the outer box (2).

2. The supplies packaging according to claim 1,
**characterized in that**
on the lid (9) and/or on the plastic waste container (13) means, preferably constructed as a snap-on connection, are provided for the non-detachable securing of the lid (9) on the plastic waste container (13) and thereby closing of the opening side (16) of the plastic waste container (13).

3. The supplies packaging according to one of the preceding claims,
**characterized in that**
the outer box (2) is contoured in a cuboid shape and/or that the plastic waste container (13) is contoured in a cuboid shape, if applicable with rounded corners.

4. The supplies packaging according to one of the preceding claims,
**characterized in that**
the medical supplies (17) comprise cannulas and/or needles and/or blades and/or are sharp-edged and/or pointed.

5. The supplies packaging according to one of the preceding claims,
**characterized in that**
the diameter and/or the contour of the effective insertion opening (21) is adapted to the dimensions of the supplies (17), in particular after removal of a protective cap, such that these are able to be inserted individually through the insertion opening (21) after their use.

6. The supplies packaging according to one of the preceding claims,
**characterized in that**
the insertion opening contour is constructed for the stripping of the supplies (17).

7. The supplies packaging according to one of the preceding claims,
**characterized in that**
means are provided for opening and/or closing the insertion opening (21) on the lid (9).

8. The supplies packaging according to claim 7,
**characterized in that**
the insertion opening (21) is surrounded by a cross-sectional contour elastically deformable by inserting of the supplies (17), which contour prevents a falling out of the inserted supplies (17) in the case of a downwardly directed insertion opening (21).

9. The supplies packaging according to one of the preceding claims,
**characterized in that**
the minimum wall thickness of the circumferential wall (14) and/or of the base of the plastic waste container (13) is at least 1.25 mm and/or is selected from a range of values between 1.3 mm and 2.0 mm.

## Revendications

1. Emballage pour produits consommables médicaux (1), comprenant un carton d'emballage (2) pouvant être ouvert au niveau d'un côté supérieur (3), dans lequel est reçue une pluralité de produits consommables médicaux (17), en particulier des canules à usage unique, un récipient de déchets en plastique (13) en forme de cuvette recevant les produits consommables médicaux (17) étant disposé dans le carton d'emballage (2) en vue de recevoir ultérieurement les produits consommables (17) après leur utilisation, lequel est tourné vers un côté inférieur (15) du carton d'emballage (2) avec un côté d'ouverture (16) pouvant être fermé au moyen d'un couvercle (9) disposé entre un côté de fond fermé (18) du récipient de déchets en plastique (13) et le côté supérieur (3) du carton d'emballage (2), présentant une ouverture d'enfichage (21) pour les produits consommables médicaux usagés (17), et vers le côté supérieur (3) du carton d'emballage (2) avec le côté de fond fermé (18).

2. Emballage pour produits consommables selon la revendication 1, **caractérisé en ce que** des moyens réalisés sur le couvercle (9) et/ou le récipient de déchets en plastique (13), de préférence en tant que connexion par encliquetage, sont prévus pour la fixation inamovible du couvercle (9) au récipient de déchets en plastique (13) et de ce fait pour la fermeture du côté d'ouverture (16) du récipient de déchets en plastique (13).

3. Emballage pour produits consommables selon l'une quelconque des revendications précédentes, **caractérisé en ce que** le carton d'emballage (2) présente un contour quadrilatéral et/ou **en ce que** le récipient de déchets en plastique (13) présente un contour quadrilatéral, éventuellement avec des coins arrondis.

4. Emballage pour produits consommables selon l'une quelconque des revendications précédentes, **caractérisé en ce que** les produits consommables médicaux (17) comprennent des canules et/ou des aiguilles et/ou des arêtes de coupe et/ou présente des arêtes vives et/ou sont pointus.

5. Emballage pour produits consommables selon l'une quelconque des revendications précédentes, **caractérisé en ce que** le diamètre et/ou le contour de l'ouverture d'enfichage efficace (21) est adapté aux dimensions des produits consommables (17), en particulier après l'enlèvement d'un capuchon de protection, de telle sorte que ceux-ci puissent être enfoncés individuellement à travers l'ouverture d'enfichage (21) après leur utilisation.

6. Emballage pour produits consommables selon l'une quelconque des revendications précédentes, **caractérisé en ce que** le contour de l'ouverture d'enfichage est réalisé pour racler les produits consommables (17).

7. Emballage pour produits consommables selon l'une quelconque des revendications précédentes, **caractérisé en ce que** des moyens sont prévus pour ouvrir et/ou fermer l'ouverture d'enfichage (21) au niveau du couvercle (9).

8. Emballage pour produits consommables selon la revendication 7, **caractérisé en ce que** l'ouverture d'enfichage (21) est entourée par un contour en section transversale élastiquement déformable par l'enfoncement des produits consommables (17), lequel contour empêche que les produits de consommation enfoncés (17) tombent lorsque l'ouverture d'enfichage (21) est orientée vers le bas.

9. Emballage pour produits consommables selon l'une quelconque des revendications précédentes, **caractérisé en ce que** l'épaisseur de paroi minimale de la paroi périphérique (14) et/ou du fond du récipient de déchets en plastique (13) est d'au moins 1,25 mm et/ou est choisie dans une plage de valeurs comprise entre 1,3 mm et 2,0 mm.
